(19) Europäisches Patentamt
European Patent Office
Office européen des brevets

(11) Veröffentlichungsnummer: **0 325 085 B1**

(12) **EUROPÄISCHE PATENTSCHRIFT**

(45) Veröffentlichungstag der Patentschrift: **18.08.93**

(21) Anmeldenummer: **88710003.0**

(22) Anmeldetag: **19.01.88**

Die Akte enthält technische Angaben, die nach dem Eingang der Anmeldung eingereicht wurden und die nicht in dieser Patentschrift enthalten sind.

(51) Int. Cl.⁵: **C07D 309/32**, C07D 409/04, C07D 409/14, C07D 407/04, A01N 43/16, A01N 43/18, A01N 43/22, A01N 43/24

# (54) Herbizide Tetrahydropyran-2,4-dionderivate.

(43) Veröffentlichungstag der Anmeldung:
**26.07.89 Patentblatt 89/30**

(45) Bekanntmachung des Hinweises auf die Patenterteilung:
**18.08.93 Patentblatt 93/33**

(84) Benannte Vertragsstaaten:
**BE CH DE FR GB IT LI NL**

(56) Entgegenhaltungen:
**EP-A- 0 164 056**
**AU-A- 560 716**
**GB-A- 2 140 803**

(73) Patentinhaber: **BASF Aktiengesellschaft**
**Carl-Bosch-Strasse 38**
**D-67063 Ludwigshafen(DE)**

(72) Erfinder: **Kast, Juergen, Dr.**
**Kastanienstrasse 24**
**D-6737 Boehl-Iggelheim(DE)**
Erfinder: **Keil, Michael, Dr.**
**Fontanestrasse 4**
**D-6713 Freinsheim(DE)**
Erfinder: **Kolassa, Dieter, Dr.**
**Moltkestrasse 8**
**D-6700 Ludwigshafen(DE)**
Erfinder: **Schirmer, Ulrich, Dr.**
**Berghalde 79**
**D-6900 Heidelberg(DE)**
Erfinder: **Wuerzer, Bruno, Dr.**
**Ruedigerstrasse 13**
**D-6701 Otterstadt(DE)**
Erfinder: **Meyer, Norbert, Dr.**
**Dossenheimer Weg 22**
**D-6802 Ladenburg(DE)**
Erfinder: **Rademacher, Wilhelm, Dr.**
**Austrasse 1**
**D-6703 Limburgerhof(DE)**
Erfinder: **Jung, Johann, Prof. Dr.**
**Hardenburgstrasse 19**
**D-6703 Limburgerhof(DE)**

EP 0 325 085 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Tetrahydropyran-2,4-dione, Verfahren zu ihrer Herstellung, herbizide und das Pflanzenwachstum regulierende Mittel, die die neuen Wirkstoffe enthalten, sowie Verfahren zur Bekämpfung von unerwünschtem Pflanzenwachstum und zur Regulierung des Pflanzenwachstums.

Es ist bekannt, daß Tetrahydro(thio)pyran-2,4-dionoximetherderivate eine herbizide Wirkung gegenüber monokotylen Pflanzen aufweisen. Beispiele hierfür sind Tetrahydropyran-2,4-dione, die in 6-Stellung aliphatische oder aromatische Substituenten tragen (GB-A-21 40 803; EP-A-01 64 056).

Es wurden nun neue Tetrahydropyran-2,4-dionderivate gefunden, die eine gute herbizide Wirkung, vorzugsweise gegen Arten aus der Familie der Gräser (Gramineen), besitzen.

Diese Tetrahydropyran-2,4-dione haben die Formel

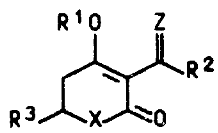 (I),

in der

- $R^1$ Wasserstoff, ein Kation, $C_1$-$C_{10}$-Alkylcarbonyl, $C_2$-$C_{10}$-Alkenylcarbonyl oder Benzoyl, das gegebenenfalls im Phenylring durch $C_1$-$C_8$-Alkyl substituiert ist,
- $R^2$ $C_1$-$C_5$-Alkyl,
- $R^3$ einen nichtaromatischen, gegebenenfalls durch $C_1$-$C_3$-Alkyl substituierten Heterocyclus mit 5 bis 7 Ringgliedern und maximal einer Doppelbindung im heterocyclischen Ring, der bis zu 2 Heteroatome aus der Gruppe Schwefel und Sauerstoff enthält,
- X Sauerstoff und
- Z Sauerstoff oder den Rest NO-$R^4$, wobei $R^4$ für $C_1$-$C_4$-Alkyl, $C_3$-$C_4$-Alkenyl, $C_3$-$C_4$-Alkinyl, $C_2$-$C_4$-Halogenalkyl, $C_2$-$C_4$-Halogenalkenyl, $C_2$-$C_3$-Alkoxyalkyl oder den Rest $CH_2$-$R^5$, in dem $R^5$ für einen 5-Ring-Heterocyclus mit 1 bis 3 Heteroatomen, wobei die Zahl der Stickstoffatome maximal drei, die Zahl der Sauerstoffatome maximal zwei und die Zahl der Schwefelatome maximal eins beträgt, mit gegebenenfalls bis zu 2 Doppelbindungen und bis zu 2 Substituenten aus der Gruppe $C_1$-$C_4$-Alkyl, $C_1$-$C_4$-Alkoxy, Halogen, Trifluormethyl, $C_1$-$C_4$-Alkoxymethyl, $C_1$-$C_4$-Alkylthiomethyl und Vinyl oder für gegebenenfalls durch bis zu 3 Substituenten aus der Gruppe $C_1$-$C_4$-Alkyl, $C_1$-$C_4$-Alkoxy, Halogen, Trifluormethyl, Nitro und $C_1$-$C_4$-Dialkylamino substituiertes Phenyl steht,

bedeuten.

Die Tetrahydropyran-2,4-dionderivate der Formel I, in der Z für den Rest NO-$R^4$ steht, besitzen eine gute herbizide Wirkung, während die Tetrahydropyran-2,4-dionderivate der Formel I, in der Z Sauerstoff bedeutet, vorzugsweise wachstumsregulierende Eigenschaften aufweisen.

Die Verbindungen der Formel I können in mehreren tautomeren Formen auftreten, die alle vom Patentanspruch umfaßt werden. Beispielsweise können die Verbindungen mit $R^1$ = H und Z = NO-$R^4$ u.a. in folgenden tautomeren Formen auftreten:

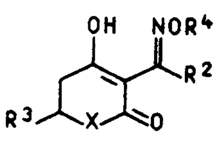 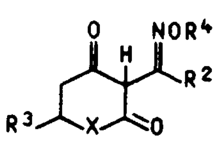 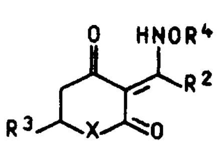 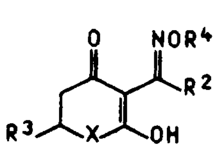

Die $C_2$-$C_4$-Halogenalkyl- oder -alkenyl für $R^4$ enthalten diese Reste 1 bis 3 Halogenatome. Für den Fall, daß $R^4$ $C_3$-$C_4$-Alkenyl oder $C_2$-$C_4$-Halogenalkenyl bedeutet, sind bei denjenigen Resten, bei denen E- und Z-Isomere auftreten können, beide Isomere vom Anspruch umfaßt.

$R^1$ in Formel I steht für Wasserstoff, für $C_1$-$C_{10}$-Alkylcarbonyl, vorzugsweise für $C_1$-$C_4$-Alkylcarbonyl, für $C_2$-$C_{10}$-Alkenylcarbonyl, vorzugsweise $C_2$-$C_4$-Alkenylcarbonyl, wie Acetyl, Propionyl, Acroyl, Butyryl, Isobutyryl, Pivaloyl, Valeroyl, 2-Ethylhexanoyl, Caproyl, Caprinoyl, Lauryl, Palmitoyl, Stearoyl, Oleoyl, für gegebenenfalls durch $C_1$-$C_8$-Alkyl, vorzugsweise $C_1$-$C_4$-Alkyl, substituiertes Benzoyl, wie 4-Methylbenzoyl, 4-Hexylbenzoyl oder Benzoyl. Bevorzugte Alkenylcarbonylreste sind solche mit nur einer C-C-Doppelbindung.

$R^2$ in Formel I steht beispielsweise für Methyl, Ethyl, n-Propyl, Isopropyl, n-Butyl, sec-Butyl, Isobutyl, t-Butyl oder Pentyl.

$R^3$ in Formel I steht für einen nicht aromatischen Heterocyclus mit 5 bis 7 Ringgliedern und maximal einer Doppelbindung. Die Zahl der Heteroatome ist begrenzt auf maximal zwei aus der Gruppe Sauerstoff und Schwefel. Diese heterocyclischen Reste können durch $C_1$-$C_3$-Alkyl substituiert sein.

$R^3$ bedeutet beispielsweise gegebenenfalls durch $C_1$-$C_3$-Alkyl substituiertes Tetrahydropyranyl, Tetrahydrothiopyranyl, 5,6-Dihydro-2H-pyranyl, 5,6-Dihydro-2H-thiopyranyl, Tetrahydrofuranyl oder 1,3-Dioxepanyl, wie Tetrahydropyran-3-yl, Tetrahydropyran-4-yl, Tetrahydrothiopyran-3-yl, Tetrahydrothiopyran-4-yl,5,6-Dihydro-2H-pyran-3-yl, 5,6-Dihydro-2H-thiopyran-3-yl, 1,4-Dioxanyl-, 5,6-Dihydro-2H-2,6-dimethylpyran-3-yl, Tetrahydrofuran-2-yl, Tetrahydrofuran-3-yl, 2-Isopropyl-1,3-dioxepan-5-yl, 2-Methyl-1,3-dioxepan-5-yl.

$R^4$ im Rest NO-$R^4$ für Z bedeutet $C_1$-$C_4$-Alkyl, $C_3$-$C_4$-Alkenyl, $C_3$-$C_4$-Alkinyl, $C_2$-$C_4$-Halogenalkyl oder $C_2$-$C_4$-Halogenalkenyl, jeweils vorzugsweise mit 1 bis 3 Halogenatomen aus der Gruppe Fluor und Chlor, oder $C_2$-$C_3$-Alkoxyalkyl, beispielsweise Methyl, Ethyl, Propyl, Allyl, (E)-But-2-en-1-yl, Propargyl, 2-Chlorallyl, (E)-3-Chlorpropen-1-yl, 3-Fluorpropyl, 2-Methoxethyl oder den Rest $CH_2$-$R^5$, wobei $R^5$ einen 5-Ring-Heterocyclus mit 1 bis 3 Heteroatomen und gegebenenfalls bis zu zwei Doppelbindungen bedeutet. Diese Heteroatome können Stickstoff-, Schwefel- oder Sauerstoffatome sein, wobei die Zahl der Stickstoffatome maximal drei, die Zahl der Sauerstoffatome maximal zwei und die Zahl der Schwefelatome maximal eins beträgt. Diese heterocyclischen Reste können bis zu zwei Substituenten aus der Gruppe $C_1$-$C_4$-Alkyl, $C_1$-$C_4$-Alkoxy, Halogen, Trifluormethyl, $C_1$-$C_4$-Alkoxymethyl, $C_1$-$C_4$-Alkylthiomethyl und Vinyl tragen. $R^5$ kann außerdem gegebenenfalls durch bis zu 3 Substituenten aus der Gruppe $C_1$-$C_4$-Alkyl, $C_1$-$C_4$-Alkoxy, Halogen, Trifluormethyl, Nitro und $C_1$-$C_4$-Dialkylamino substituiertes Phenyl bedeuten. Beispiele für $R^5$ sind p-Fluorphenyl, Phenyl, Thien-2-yl, m-Trifluormethylphenyl, p-Nitrophenyl, 5-Chlorthien-2-yl.

Als Salze der Verbindungen der Formel I ($R^1$ = Kation), kommen landwirtschaftlich brauchbare Salze, beispielsweise Alkalimetallsalze, insbesondere Kalium- oder Natriumsalze, Erdalkalimetallsalze, insbesondere Calcium- und Mangan-, Kupfer-, Zink- oder Eisensalze, Ammonium-, Sulfonium-, Sulfoxonium- und Phosphoniumsalze, beispielsweise Ammonium-Tetraalkylammonium-, Benzyltrialkylammonium-, Trialkylsulfoxonium- oder Trialkylsulfoniumsalze in Betracht.

Bevorzugt sind Tetrahydropyran-2,4-dionderivate der Formel I, bei denen $R^2$ $C_2$-$C_3$-Alkyl bedeutet.

Die Tetrahydropyran-2,4-dionderivate der Formel I, in der Z den Rest $NOR^4$ bedeutet, können durch Umsetzung derjenigen Tetrahydropyran-2,4-dionderivate der Formel I, in der Z Sauerstoff bedeutet, mit einer Ammoniumverbindung der Formel $[R^4O\text{-}NH_3]$ Y, in der $R^4$ die obengenannten Bedeutungen hat und Y ein Anion (Chlorid, Bromid oder Sulfat) bedeutet, erhalten werden.

Zweckmäßigerweise führt man die Reaktion in heterogener Phase in einem inerten Lösungsmittel bei einer Temperatur zwischen 0 und 80°C bzw. zwischen 0°C und dem Siedepunkt des Reaktionsgemisches in Gegenwart einer Base durch. Geeignete Basen sind z.B. Carbonate, Hydrogencarbonate, Acetate, Alkoholate, Hydroxide oder Oxide von Alkali- oder Erdalkalimetallen, insbesondere Natriumhydroxid, Kaliumhydroxid, Magnesiumoxid, Calciumoxid. Außerdem können organische Basen, wie Pyridin oder tertiäre Amine, Verwendung finden. Die Base wird beispielsweise in Mengen von 0,5 bis 2 Mol, bezogen auf die Ammoniumverbindung, zugesetzt (DE-A-34 33 767).

Als Lösungsmittel sind beispielsweise Dimethylsulfoxid; Alkohole, wie Methanol, Ethanol oder Isopropanol; Toluol; gegebenenfalls chlorierte Kohlenwasserstoffe, wie Chloroform, Dichlormethan, Hexan oder Cyclohexan; Ester, wie Essigsäureethylester; oder Ether, wie Dioxan oder Tetrahydrofuran, geeignet.

Die Tetrahydropyran-2,4-dionderivate der Formel I, in denen Z Sauerstoff bedeutet, erhält man in an sich bekannter Weise durch Umsetzung von Tetrahydropyran-2,4-dionderivaten der Formel I

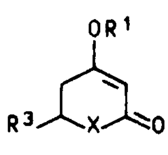

(II),

in der $R^1$ Wasserstoff bedeutet und $R^3$ und X die obengenannten Bedeutungen haben, mit Säurechloriden der Formel $R^2$-COCl, in denen $R^2$ die obengenannten Bedeutungen hat, in Gegenwart einer Base (z.B. Triethylamin) in einem inerten Verdünnungsmittel (z.B. Tetrahydrofuran) zum Enolester und anschließender Behandlung mit einem sauren oder basischen Katalysator in einem inerten Lösungsmittel (z.B. Essigester, Toluol) bei Temperaturen zwischen 0 und 100°C (JP-A-63052/79). Beispiele für saure oder basische Katalysatoren sind Aluminiumchlorid, Eisenchlorid, Imidazolderivate, z.B. Imidazol, oder Pyridinderivate, z.B. Dimethylaminopyridin.

Zur Herstellung der Verbindungen der Formel II gibt es mehrere literaturbekannte Verfahren:

a) Umsetzung einer 4-Brom-3-methoxy-crotonsäureesters der Formel III, in der R für Methyl, Ethyl oder Propyl steht, mit einem Aldehyd der Formel $R^3$-CHO, in der $R^3$ die obengenannten Bedeutungen hat, in einem inerten Lösungsmittel (z.B. Tetrahydrofuran, Benzol) in Gegenwart von Zink (J. Heterocyclic Chem. 21, 1755 (1984)).

b) Umsetzung von Diketen mit einem Aldehyd der Formel $R^3$-CHO, in der $R^3$ die obengenannten Bedeutungen hat, in einem inerten Lösungsmittel (z.B. Dichlormethan) mit einer Lewissäure (z.B. Titantetrachlorid) als Katalysator (Chem. Letters, 1975, 101).

c) Umsetzung des Dianions eines Acetessigsäureesters der Formel IV, in der R für Methyl, Ethyl oder Propyl steht, mit einem Aldehyd der Formel $R^3$-CHO, in der $R^3$ die obengenannten Bedeutungen hat, in einem inerten Lösungsmittel, z.B. Tetrahydrofuran (Angew. Chem. 86, 40 (1974)).

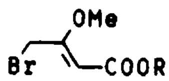

(III)

$CH_3-CO-CH_2-COOR$

(IV)

Die aufgeführten $\alpha,\beta$-ungesättigten Verbindungen der Formel III können sowohl als cis- wie auch als trans-Isomere vorliegen.

Zu den Aldehyden der Formel $R^3$-CHO gelangt man nach literaturbekannten Verfahren, z.B. durch Oxidation von Alkoholen, Reduktion von Carbonsäurederivaten oder Hydroformylierung von Olefinen.

Herstellungsbeispiel

a) 5-Hydroxy-3-oxo-5-(tetrahydrothiopyran-3-yl)-pentansäuremethylester

6,6 g Natriumhydrid (80 %) werden in 400 ml Tetrahydrofuran vorgelegt und auf 0 °C abgekühlt. Unter Beibehaltung dieser Temperatur werden 24,4 g Acetessigsäuremethylester zugetropft, 15 Minuten nachgerührt, anschließend 131,5 ml einer 1,6 molaren Butyllithiumlösung in Hexan zugetropft und 30 Minuten nachgerührt. Bei 0 bis 3 °C werden 26 g Tetrahydro-thiopyran-3-carbaldehyd zugetropft und zur Vervollständigung der Reaktion weitere 30 Minuten gerührt. Dazu werden vorsichtig 45 ml konzentrierte Salzsäure zugetropft. Das kalte Reaktionsgemisch wird in 250 ml Eiswasser gegossen und die organische Phase abgetrennt. Die wäßrige Phase wird zweimal mit je 250 ml Methyltertiärbutylether extrahiert. Die vereinigten organischen Extrakte werden einmal mit 300 ml gesättigter Natriumchloridlösung gewaschen, mit Natriumsulfat getrocknet und einrotiert. Es werden 48,1 g Öl erhalten, das sofort weiter umgesetzt wird.

b) 5-(Tetrahydrothiopyran-3-yl)-tetrahydropyran-2,4-dion

48 g 5-Hydroxy-3-oxo-5-(tetrahyrothiopyran-3-yl)-pentansäuremethylester werden in 300 ml 5 %iger Natronlauge 16 Stunden gerührt. Zur Reinigung wird die wäßrige Lösung zweimal mit je 250 ml Toluol extrahiert. Die Wasserphase wird unter Eiskühlung mit konzentrierter Salzsäure auf pH 1 angesäuert. Der ausgefallene Niederschlag wird abgesaugt und mit Ether verrührt. Der Rückstand wird abgesaugt und getrocknet. Man erhält 27 g Feststoff, der bei 91 bis 93 °C schmilzt.

c) 3-Propionyl-5-(tetrahydrothiopyran-3-yl)-tetrahydropyran-2,4-dion

5 g 5-(Tetrahydrothiopyran-3-yl)-tetrahydropyran-2,4-dion werden in 50 ml trockenem Tetrahydrofuran vorgelegt und mit 2,4 g Triethylamin versetzt. Bei 0 °C werde 2,2 g Propionsäurechlorid zugetropft und anschließend 12 Stunden bei Raumtemperatur gerührt. Zur Aufarbeitung wird mit 150 ml Wasser verdünnt, die wäßrige Phase abgetrennt und mit 100 ml Ether extrahiert. Die vereinigten organischen Phasen werden je einmal mit eiskalter 5 %iger Salzsäure und Wasser gewaschen. Nach dem Trocknen mit Magnesiumsulfat wird das Lösungsmittel entfernt. Der ölige Rückstand wird in 50 ml trockenem Toluol gelöst, mit 0,3 g 3-Dimethylaminopyridin versetzt und 2 h bei 80 °C gerührt. Nach dem Abkühlen wird zweimal mit je 25 ml 2 %iger Salzsäure und zweimal mit je 25 ml Wasser extrahiert. Die organische Phase wird über Natriumsulfat getrocknet und einrotiert. Der Rückstand wird mit Methylenchlorid an Kieselgel chromatographiert. Es werden 1,8 g der gewünschten Verbindung erhalten.

d) 3-(1-Ethoxyiminopropyl)-5-(tetrahydrothiopyran-3-yl)-tetrahydropyran-2,4-dion (Verbindung Nr. 36 in Tabelle 1)

1,6 g 3-Propionyl-5-(tetrahydrothiopyran-3-yl)-tetrahydropyran-2,4-dion werden in 25 ml trockenem Methanol gelöst und nacheinander mit 0,6 g Natriumbicarbonat und 0,7 g Ethoxyaminhydrochlorid versetzt. Das Reaktionsgemisch wird 12 Stunden bei Raumtemperatur gerührt und anschließend zur Trockene

eingedampft. Der Rückstand wird in 50 ml Methylenchlorid aufgenommen, einmal mit Wasser, zweimal mit gesättigter Natriumhydrogencarbonatlösung und nochmals mit Wasser gewaschen. Die organische Phase wird mit Natriumsulfat getrocknet und das Lösungsmittel abgezogen. Es werden 0,8 g Oximether erhalten.

In analoger Weise lassen sich die in den folgenden Tabellen genannten Verbindungen der Formel I herstellen.

Tabelle 1

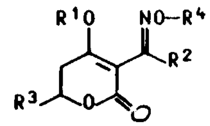

(Ia)

| Nr. | $R^3$ | $R^2$ | $R^1$ | $R^4$ | phys. Daten |
|---|---|---|---|---|---|
| 1 | Tetrahydropyran-3-yl | Ethyl | H | Ethyl | |
| 2 | Tetrahydropyran-3-yl | Ethyl | H | Allyl | |
| 3 | Tetrahydropyran-3-yl | Ethyl | H | (E)-3-Chlorallyl | |
| 4 | Tetrahydropyran-3-yl | Ethyl | H | (E)-2-Butenyl | |
| 5 | Tetrahydropyran-3-yl | Ethyl | H | Thien-2-ylmethyl | |
| 6 | Tetrahydropyran-3-yl | Ethyl | H | 5-Chlorthien-2-ylmethyl | |
| 7 | Tetrahydropyran-3-yl | Ethyl | H | p-Fluorphenylmethyl | |
| 8 | Tetrahydropyran-3-yl | Ethyl | H | m-Trifluormethylphenylmethyl | |
| 9 | Tetrahydropyran-3-yl | Ethyl | H | p-Nitrophenylmethyl | |
| 10 | Tetrahydropyran-3-yl | Ethyl | Acetyl | Allyl | |
| 11 | Tetrahydropyran-3-yl | Ethyl | Acetyl | (E)-3-Chlorallyl | |
| 12 | Tetrahydropyran-3-yl | Ethyl | Acetyl | p-Fluorphenylmethyl | |
| 13 | Tetrahydropyran-3-yl | Ethyl | Benzoyl | Allyl | |
| 14 | Tetrahydropyran-3-yl | Ethyl | Benzoyl | (E)-3-Chlorallyl | |
| 15 | Tetrahydropyran-3-yl | Ethyl | Benzoyl | m-Trifluormethylphenylmethyl | |
| 16 | Tetrahydropyran-3-yl | Propyl | H | Ethyl | NMR: 4,1-4,3, m; 4,05,-4,2, q; 3,8-4,0, m; 1,25-1,4, t; 0,8-1,1, t; |

Tabelle 1 (Fortsetzung)

| Nr. | $R^3$ | $R^2$ | $R^1$ | $R^4$ | phys. Daten |
|---|---|---|---|---|---|
| 17 | Tetrahydropyran-3-yl | Propyl | H | Allyl | |
| 18 | Tetrahydropyran-3-yl | Propyl | H | (E)-3-Chlorallyl | |
| 19 | Tetrahydropyran-3-yl | Propyl | H | (E)-2-Butenyl | |
| 20 | Tetrahydropyran-3-yl | Propyl | H | Thien-2-ylmethyl | |
| 21 | Tetrahydropyran-3-yl | Propyl | H | 5-Chlorthien-2-ylmethyl | |
| 22 | Tetrahydropyran-3-yl | Propyl | H | p-Fluorphenylmethyl | |
| 23 | Tetrahydropyran-3-yl | Propyl | H | m-Trifluormethylphenylmethyl | |
| 24 | Tetrahydropyran-3-yl | Propyl | H | p-Nitrophenylmethyl | |
| 25 | Tetrahydropyran-3-yl | Propyl | Acetyl | Ethyl | |
| 26 | Tetrahydropyran-3-yl | Propyl | Acetyl | Allyl | |
| 27 | Tetrahydropyran-3-yl | Propyl | Acetyl | Methoxymethyl | |
| 28 | Tetrahydropyran-3-yl | Propyl | Benzoyl | Ethyl | |
| 29 | Tetrahydropyran-3-yl | Propyl | Benzoyl | Allyl | |
| 30 | Tetrahydropyran-3-yl | Propyl | Benzoyl | 2-Methoxyethyl | |
| 31 | Tetrahydropyran-3-yl | Pentyl | H | Ethyl | |
| 32 | Tetrahydropyran-3-yl | Pentyl | H | Allyl | |
| 33 | Tetrahydropyran-3-yl | Pentyl | H | (E)-3-Chlorallyl | |
| 34 | Tetrahydropyran-3-yl | Pentyl | Acetyl | Ethyl | |
| 35 | Tetrahydropyran-3-yl | Pentyl | Benzoyl | Ethyl | |
| 36 | Tetrahydrothiopyran-3-yl | Ethyl | H | Ethyl | NMR: 1, 19, t; 1, 36, t; 4, 13, q; 4, 2-4, 3, m; |
| 37 | Tetrahydrothiopyran-3-yl | Ethyl | H | Allyl | NMR: 1, 19, t; 4, 15-4, 3, m; 4, 56, d; 5, 3-5, 5, m; 5, 9-6, 1, m; |
| 38 | Tetrahydrothiopyran-3-yl | Ethyl | H | (E)-3-Chlorallyl | NMR: 1, 1-1, 2, d; 6, 0-6, 4, m; 4, 45-4, 55, d; 4, 15-4, 3, m; 1, 1-1, 2, d |

Tabelle 1 (Fortsetzung)

| Nr. | $R^3$ | $R^2$ | $R^1$ | $R^4$ | phys. Daten |
|---|---|---|---|---|---|
| 39 | Tetrahydrothiopyran-3-yl | Ethyl | H | (E)-2-Butenyl | |
| 40 | Tetrahydrothiopyran-3-yl | Ethyl | H | Thien-2-ylmethyl | |
| 41 | Tetrahydrothiopyran-3-yl | Ethyl | H | 5-Chlorthien-2-ylmethyl | |
| 42 | Tetrahydrothiopyran-3-yl | Ethyl | H | p-Fluorphenylmethyl | |
| 43 | Tetrahydrothiopyran-3-yl | Ethyl | H | m-Trifluormethylphenylmethyl | |
| 44 | Tetrahydrothiopyran-3-yl | Ethyl | H | p-Nitrophenylmethyl | |
| 45 | Tetrahydrothiopyran-3-yl | Ethyl | Acetyl | Allyl | |
| 46 | Tetrahydrothiopyran-3-yl | Ethyl | Acetyl | (E)-3-Chlorallyl | |
| 47 | Tetrahydrothiopyran-3-yl | Ethyl | Acetyl | p-Fluorphenylmethyl | |
| 48 | Tetrahydrothiopyran-3-yl | Ethyl | Benzoyl | Allyl | |
| 49 | Tetrahydrothiopyran-3-yl | Ethyl | Benzoyl | (E)-3-Chlorallyl | |
| 50 | Tetrahydrothiopyran-3-yl | Ethyl | Benzoyl | m-Trifluormethylphenylmethyl | |
| 51 | Tetrahydrothiopyran-3-yl | Propyl | H | Ethyl | NMR: 4,1-4,3, m; 4,0-4,2, q; 2,95-3,1, m; 1,3-1,4, t; 0,9-1,1, t |
| 52 | Tetrahydrothiopyran-3-yl | Propyl | H | Allyl | NMR: 0,98, t; 4,1-4,3, m; 4,55, d; 5,2-5,5, m; 5,9-6,1, m; |
| 53 | Tetrahydrothiopyran-3-yl | Propyl | H | (E)-3-Chlorallyl | NMR: 0,98, t; 4,1-4,3, m; 4,53, d; 6,0-6,2, m; 6,3-6,45, d; |
| 54 | Tetrahydrothiopyran-3-yl | Propyl | H | (E)-2-Butenyl | NMR: 1,0, t; 1,8, d; 4,1-4,3, m; 4,45, d; 5,5-5,7, m; 5,8-6,0, m; |
| 55 | Tetrahydrothiopyran-3-yl | Propyl | H | Thien-2-ylmethyl | NMR: 0,95, t; 4,1-4,3, m; 5,2, s; 7,04, dd; 7,12, d; 7,37, d; |
| 56 | Tetrahydrothiopyran-3-yl | Propyl | H | 5-Chlorthien-2-ylmethyl | NMR: 0,99, t; 4,15-4,3, m; 5,1, s; 6,8-6,9, m; |

Tabelle 1 (Fortsetzung)

| Nr. | $R^3$ | $R^2$ | $R^1$ | $R^4$ | phys. Daten |
|---|---|---|---|---|---|
| 57 | Tetrahydrothiopyran-3-yl | Propyl | H | p-Fluorphenylmethyl | |
| 58 | Tetrahydrothiopyran-3-yl | Propyl | H | m-Trifluormethylphenylmethyl | NMR: 0,97, t; 4,1-4,25, m; 5,12, dd; 7,4-7,7, m; |
| 59 | Tetrahydrothiopyran-3-yl | Propyl | H | p-Nitrophenylmethyl | |
| 60 | Tetrahydrothiopyran-3-yl | Propyl | Acetyl | Ethyl | |
| 61 | Tetrahydrothiopyran-3-yl | Propyl | Acetyl | Allyl | |
| 62 | Tetrahydrothiopyran-3-yl | Propyl | Acetyl | 3-Fluorpropyl | |
| 63 | Tetrahydrothiopyran-3-yl | Propyl | Benzoyl | Ethyl | |
| 64 | Tetrahydrothiopyran-3-yl | Propyl | Benzoyl | Allyl | |
| 65 | Tetrahydrothiopyran-3-yl | Propyl | Benzoyl | Phenylmethyl | |
| 66 | Tetrahydrothiopyran-3-yl | Pentyl | H | Ethyl | |
| 67 | Tetrahydrothiopyran-3-yl | Pentyl | H | Allyl | |
| 68 | Tetrahydrothiopyran-3-yl | Pentyl | H | (E)-3-Chlorallyl | |
| 69 | Tetrahydrothiopyran-3-yl | Pentyl | Acetyl | Ethyl | |
| 70 | Tetrahydrothiopyran-3-yl | Pentyl | Benzoyl | Ethyl | |
| 71 | Tetrahydropyran-4-yl | Ethyl | H | Ethyl | NMR: 1,21, t; 1,33, t; 3,4, dd; 3,95-4,25, m; |
| 72 | Tetrahydropyran-4-yl | Ethyl | H | Allyl | |
| 73 | Tetrahydropyran-4-yl | Ethyl | H | (E)-3-Chlorallyl | |
| 74 | Tetrahydropyran-4-yl | Ethyl | H | (E)-2-Butenyl | |
| 75 | Tetrahydropyran-4-yl | Ethyl | H | 5-Chlorthien-2-yl | |
| 76 | Tetrahydropyran-4-yl | Ethyl | H | m-Trifluormethylphenylmethyl | NMR: 1,19, t; 2,8-3,1, m; 3,3-3,5, m; 3,9-4,2, m; 5,15, s; 7,4-7,75, m; |
| 77 | Tetrahydropyran-4-yl | Ethyl | Acetyl | Allyl | |
| 78 | Tetrahydropyran-4-yl | Ethyl | Benzoyl | Allyl | |

Tabelle 1 (Fortsetzung)

| Nr. | $R^3$ | $R^2$ | $R^1$ | $R^4$ | phys. Daten |
|---|---|---|---|---|---|
| 79 | Tetrahydropyran-4-yl | Propyl | H | Ethyl | |
| 80 | Tetrahydropyran-4-yl | Propyl | H | Allyl | |
| 81 | Tetrahydropyran-4-yl | Propyl | H | (E)-3-Chlorallyl | |
| 82 | Tetrahydropyran-4-yl | Propyl | H | 5-Chlorthien-2-ylmethyl | |
| 83 | Tetrahydropyran-4-yl | Propyl | H | p-Fluorphenylmethyl | |
| 84 | Tetrahydropyran-4-yl | Propyl | Acetyl | Ethyl | |
| 85 | Tetrahydropyran-4-yl | Propyl | Benzoyl | Ethyl | |
| 86 | Tetrahydrothiopyran-4-yl | Ethyl | H | Allyl | |
| 87 | Tetrahydrothiopyran-4-yl | Ethyl | H | (E)-3-Chlorallyl | |
| 88 | Tetrahydrothiopyran-4-yl | Ethyl | H | 3-Fluorpropyl | |
| 89 | Tetrahydrothiopyran-4-yl | Ethyl | H | 5-Chlorthien-2-ylmethyl | |
| 90 | Tetrahydrothiopyran-4-yl | Ethyl | H | p-Nitrophenylmethyl | |
| 91 | Tetrahydrothiopyran-4-yl | Ethyl | Acetyl | Allyl | |
| 92 | Tetrahydrothiopyran-4-yl | Ethyl | Benzoyl | Allyl | |
| 93 | Tetrahydrothiopyran-4-yl | Propyl | H | Ethyl | |
| 94 | Tetrahydrothiopyran-4-yl | Propyl | H | Allyl | |
| 95 | Tetrahydrothiopyran-4-yl | Propyl | H | (E)-3-Chlorallyl | |
| 96 | Tetrahydrothiopyran-4-yl | Propyl | H | p-Fluorphenylmethyl | |
| 97 | Tetrahydrothiopyran-4-yl | Propyl | H | p-Nitrophenylmethyl | |
| 98 | Tetrahydrothiopyran-4-yl | Propyl | Acetyl | Ethyl | |
| 99 | Tetrahydrothiopyran-4-yl | Propyl | Benzoyl | Ethyl | |
| 100 | 5,6-Dihydro-2H-pyran-3-yl | Ethyl | H | Allyl | |
| 101 | 5,6-Dihydro-2H-pyran-3-yl | Ethyl | H | (E)-3-Chlorallyl | |

Tabelle 1 (Fortsetzung)

| Nr. | $R^3$ | $R^2$ | $R^1$ | $R^4$ | phys. Daten |
|---|---|---|---|---|---|
| 102 | 5,6-Dihydro-2H-pyran-3-yl | Ethyl | H | (E)-2-Butenyl | |
| 103 | 5,6-Dihydro-2H-pyran-3-yl | Ethyl | H | Phenylmethyl | |
| 104 | 5,6-Dihydro-2H-pyran-3-yl | Ethyl | Acetyl | Allyl | |
| 105 | 5,6-Dihydro-2H-pyran-3-yl | Ethyl | Benzoyl | Allyl | |
| 106 | 5,6-Dihydro-2H-pyran-3-yl | Propyl | H | Ethyl | |
| 107 | 5,6-Dihydro-2H-pyran-3-yl | Propyl | H | Allyl | |
| 108 | 5,6-Dihydro-2H-pyran-3-yl | Propyl | H | (E)-3-Chlorallyl | |
| 109 | 5,6-Dihydro-2H-pyran-3-yl | Propyl | H | 2-Chlorallyl | |
| 110 | 5,6-Dihydro-2H-pyran-3-yl | Propyl | Acetyl | Ethyl | |
| 111 | 5,6-Dihydro-2H-pyran-3-yl | Propyl | Benzoyl | Ethyl | |
| 112 | 5,6-Dihydro-2H-thiopyran-3-yl | Ethyl | H | Allyl | |
| 113 | 5,6-Dihydro-2H-thiopyran-3-yl | Ethyl | H | (E)-3-Chlorallyl | |
| 114 | 5,6-Dihydro-2H-thiopyran-3-yl | Ethyl | H | (E)-2-Butenyl | |
| 115 | 5,6-Dihydro-2H-thiopyran-3-yl | Ethyl | H | 5-Chlorthien-2-ylmethyl | |
| 116 | 5,6-Dihydro-2H-thiopyran-3-yl | Ethyl | Acetyl | (E)-3-Chlorallyl | |
| 117 | 5,6-Dihydro-2H-thiopyran-3-yl | Ethyl | Benzoyl | (E)-3-Chlorallyl | |
| 118 | 5,6-Dihydro-2H-thiopyran-3-yl | Propyl | H | Ethyl | |
| 119 | 5,6-Dihydro-2H-thiopyran-3-yl | Propyl | H | Allyl | |
| 120 | 5,6-Dihydro-2H-thiopyran-3-yl | Propyl | H | (E)-3-Chlorallyl | |
| 121 | 5,6-Dihydro-2H-thiopyran-3-yl | Propyl | H | 5-Chlorthien-2-ylmethyl | |
| 122 | 5,6-Dihydro-2H-thiopyran-3-yl | Propyl | Acetyl | Ethyl | |
| 123 | 5,6-Dihydro-2H-thiopyran-3-yl | Propyl | Benzoyl | Ethyl | |
| 124 | 5,6-Dihydro-2,6-dimethyl-2H-pyran-3-yl | Ethyl | H | Ethyl | |

Tabelle 1 (Fortsetzung)

| Nr. | $R^3$ | $R^2$ | $R^1$ | $R^4$ | phys. Daten |
|---|---|---|---|---|---|
| 125 | 5,6-Dihydro-2,6-dimethyl-2H-pyran-3-yl | Ethyl | H | Allyl | |
| 126 | 5,6-Dihydro-2,6-dimethyl-2H-pyran-3-yl | Ethyl | H | (E)-3-Chlorallyl | |
| 127 | 5,6-Dihydro-2,6-dimethyl-2H-pyran-3-yl | Ethyl | H | (E)-2-Butenyl | |
| 128 | 5,6-Dihydro-2,6-dimethyl-2H-pyran-3-yl | Ethyl | H | p-Fluorphenylmethyl | |
| 129 | 5,6-Dihydro-2,6-dimethyl-2H-pyran-3-yl | Ethyl | H | p-Nitrophenylmethyl | |
| 130 | 5,6-Dihydro-2,6-dimethyl-2H-pyran-3-yl | Ethyl | Acetyl | (E)-3-Chlorallyl | |
| 131 | 5,6-Dihydro-2,6-dimethyl-2H-pyran-3-yl | Ethyl | Benzoyl | (E)-3-Chlorallyl | |
| 132 | 5,6-Dihydro-2,6-dimethyl-2H-pyran-3-yl | Propyl | H | Ethyl | |
| 133 | 5,6-Dihydro-2,6-dimethyl-2H-pyran-3-yl | Propyl | H | Allyl | |
| 134 | 5,6-Dihydro-2,6-dimethyl-2H-pyran-3-yl | Propyl | H | (E)-3-Chlorallyl | |
| 135 | 5,6-Dihydro-2,6-dimethyl-2H-pyran-3-yl | Propyl | H | (E)-2-Butenyl | |
| 136 | 5,6-Dihydro-2,6-dimethyl-2H-pyran-3-yl | Propyl | H | p-Fluorphenylmethyl | |
| 137 | 5,6-Dihydro-2,6-dimethyl-2H-pyran-3-yl | Propyl | H | p-Nitrophenylmethyl | |
| 138 | 5,6-Dihydro-2,6-dimethyl-2H-pyran-3-yl | Propyl | Acetyl | Ethyl | |
| 139 | 5,6-Dihydro-2,6-dimethyl-2H-pyran-3-yl | Propyl | Benzoyl | Ethyl | |
| 140 | 1,4-Dioxanyl | Ethyl | H | Allyl | |
| 141 | 1,4-Dioxanyl | Ethyl | H | (E)-3-Chlorallyl | |
| 142 | 1,4-Dioxanyl | Ethyl | H | (E)-2-Butenyl | |
| 143 | 1,4-Dioxanyl | Ethyl | H | 5-Chlorthien-2-ylmethyl | |
| 144 | 1,4-Dioxanyl | Propyl | H | Ethyl | |
| 145 | 1,4-Dioxanyl | Propyl | H | Allyl | |
| 146 | 1,4-Dioxanyl | Propyl | H | (E)-3-Chlorallyl | |
| 147 | 1,4-Dioxanyl | Propyl | H | 5-Chlorthien-2-ylmethyl | |

Tabelle 1 (Fortsetzung)

| Nr. | $R^3$ | $R^2$ | $R^1$ | $R^4$ | phys. Daten |
|---|---|---|---|---|---|
| 148 | Tetrahydrofuran-2-yl | Ethyl | H | Allyl | |
| 149 | Tetrahydrofuran-2-yl | Ethyl | H | (E)-3-Chlorallyl | |
| 150 | Tetrahydrofuran-2-yl | Ethyl | H | (E)-2-Butenyl | |
| 151 | Tetrahydrofuran-2-yl | Ethyl | H | p-Nitrophenylmethyl | |
| 152 | Tetrahydrofuran-2-yl | Propyl | H | Ethyl | |
| 153 | Tetrahydrofuran-2-yl | Propyl | H | Allyl | |
| 154 | Tetrahydrofuran-2-yl | Propyl | H | (E)-3-Chlorallyl | |
| 155 | Tetrahydrofuran-2-yl | Propyl | H | p-Nitrophenylmethyl | |
| 156 | Tetrahydrofuran-3-yl | Ethyl | H | Allyl | |
| 157 | Tetrahydrofuran-3-yl | Ethyl | H | (E)-3-Chlorallyl | |
| 158 | Tetrahydrofuran-3-yl | Ethyl | H | (E)-2-Butenyl | |
| 159 | Tetrahydrofuran-3-yl | Ethyl | H | p-Fluorphenylmethyl | |
| 160 | Tetrahydrofuran-3-yl | Propyl | H | Ethyl | |
| 161 | Tetrahydrofuran-3-yl | Propyl | H | Allyl | |
| 162 | Tetrahydrofuran-3-yl | Propyl | H | (E)-3-Chlorallyl | |
| 163 | Tetrahydrofuran-3-yl | Propyl | H | p-Fluorphenylmethyl | |
| 164 | 2-Isopropyl-1,3-dioxepan-5-yl | Ethyl | H | Ethyl | |
| 165 | 2-Isopropyl-1,3-dioxepan-5-yl | Ethyl | H | Allyl | |
| 166 | 2-Isopropyl-1,3-dioxepan-5-yl | Ethyl | H | (E)-3-Chlorallyl | |
| 167 | 2-Isopropyl-1,3-dioxepan-5-yl | Ethyl | H | (E)-2-Butenyl | |
| 168 | 2-Isopropyl-1,3-dioxepan-5-yl | Ethyl | H | p-Fluorphenylmethyl | |
| 169 | 2-Isopropyl-1,3-dioxepan-5-yl | Ethyl | H | p-Nitrophenylmethyl | |
| 170 | 2-Isopropyl-1,3-dioxepan-5-yl | Ethyl | H | 5-Chlorthien-2-ylmethyl | |

Tabelle 1 (Fortsetzung)

| Nr. | $R^3$ | $R^2$ | $R^1$ | $R^4$ | phys. Daten |
|---|---|---|---|---|---|
| 171 | 2-Isopropyl-1,3-dioxepan-5-yl | Ethyl | H | m-Trifluormethylphenylmethyl | |
| 172 | 2-Isopropyl-1,3-dioxepan-5-yl | Propyl | H | Ethyl | |
| 173 | 2-Isopropyl-1,3-dioxepan-5-yl | Propyl | H | Allyl | |
| 174 | 2-Isopropyl-1,3-dioxepan-5-yl | Propyl | H | (E)-3-Chlorallyl | |
| 175 | 2-Isopropyl-1,3-dioxepan-5-yl | Propyl | H | (E)-2-Butenyl | |
| 176 | 2-Isopropyl-1,3-dioxepan-5-yl | Propyl | H | p-Fluorphenylmethyl | |
| 177 | 2-Isopropyl-1,3-dioxepan-5-yl | Propyl | H | p-Nitrophenylmethyl | |
| 178 | 2-Isopropyl-1,3-dioxepan-5-yl | Propyl | H | 5-Chlorthien-2-ylmethyl | |
| 179 | 2-Isopropyl-1,3-dioxepan-5-yl | Propyl | H | m-Trifluormethylphenylmethyl | |
| 180 | 2-Methyl-1,3-dioxepan-5-yl | Ethyl | H | Ethyl | |
| 181 | 2-Methyl-1,3-dioxepan-5-yl | Ethyl | H | Allyl | |
| 182 | 2-Methyl-1,3-dioxepan-5-yl | Ethyl | H | (E)-3-Chlorallyl | |
| 183 | 2-Methyl-1,3-dioxepan-5-yl | Ethyl | H | (E)-2-Butenyl | |
| 184 | 2-Methyl-1,3-dioxepan-5-yl | Ethyl | H | p-Fluorphenylmethyl | |
| 185 | 2-Methyl-1,3-dioxepan-5-yl | Ethyl | H | p-Nitrophenylmethyl | |
| 186 | 2-Methyl-1,3-dioxepan-5-yl | Propyl | H | Ethyl | |
| 187 | 2-Methyl-1,3-dioxepan-5-yl | Propyl | H | Allyl | |
| 188 | 2-Methyl-1,3-dioxepan-5-yl | Propyl | H | (E)-3-Chlorallyl | |
| 189 | 2-Methyl-1,3-dioxepan-5-yl | Propyl | H | (E)-2-Butenyl | |
| 190 | 2-Methyl-1,3-dioxepan-5-yl | Propyl | H | p-Fluorphenylmethyl | |
| 191 | 2-Methyl-1,3-dioxepan-5-yl | Propyl | H | p-Nitrophenylmethyl | |

Tabelle 2

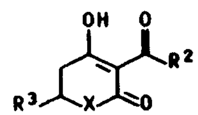

(Ic)

| Nr. | $R^3$ | $R^2$ | X | phys. Daten |
|---|---|---|---|---|
| 192 | Tetrahydropyran-3-yl | Ethyl | O | |
| 193 | Tetrahydropyran-3-yl | Propyl | O | NMR: 4,1-4,4 m; 3,8-4,0 m; 2,95-3,1 q, 0,9-1,1 t |
| 194 | Tetrahydropyran-3-yl | Pentyl | O | |
| 195 | Tetrahydrothiopyran-3-yl | Ethyl | O | Fp. 78-79°C |
| 196 | Tetrahydrothiopyran-3-yl | Propyl | O | NMR: 4,2-4,3 m; 2,9-3,0 q; 0,9-1,0 t |
| 197 | Tetrahydrothiopyran-3-yl | Pentyl | O | |
| 198 | Tetrahydropyran-4-yl | Ethyl | O | NMR: 1,22, m; 1,82-2,07, m; 2,88-3,28, m; 3,29-3,57, m; 3,9-4,4, m; |
| 199 | Tetrahydropyran-4-yl | Propyl | O | |
| 200 | Tetrahydrothiopyran-4-yl | Ethyl | O | |
| 201 | Tetrahydrothiopyran-4-yl | Propyl | O | |
| 202 | 5,6-Dihydro-2H-pyran-3-yl | Ethyl | O | NMR: 1,2, m; 2,83-3,28, m; 4,7-4,92, m; 5,85-6,06, m; |
| 203 | 5,6-Dihydro-2H-pyran-3-yl | Propyl | O | |

Tabelle 2 (Fortsetzung)

| Nr. | $R^3$ | $R^2$ | X | phys. Daten |
|---|---|---|---|---|
| 204 | 5,6-Dihydro-2H-thiopyran-3-yl | Ethyl | O | |
| 205 | 5,6-Dihydro-2H-thiopyran-3-yl | Propyl | O | |
| 206 | 5,6-Dihydro-2,6-dimethyl-2H-pyran-3-yl | Ethyl | O | |
| 207 | 5,6-Dihydro-2,6-dimethyl-2H-pyran-3-yl | Propyl | O | |
| 208 | 1,4-Dioxanyl | Ethyl | O | |
| 209 | 1,4-Dioxanyl | Propyl | O | |
| 210 | Tetrahydrofuran-2-yl | Ethyl | O | |
| 211 | Tetrahydrofuran-2-yl | Propyl | O | |
| 212 | Tetrahydrofuran-3-yl | Ethyl | O | |
| 213 | Tetrahydrofuran-3-yl | Propyl | O | |
| 214 | 2-Isopropyl-1,3-dioxepan-5-yl | Ethyl | O | |

Tabelle 2 (Fortsetzung)

| Nr. | $R^3$ | $R^2$ | X | phys. Daten |
|---|---|---|---|---|
| 215 | 2-Isopropyl-1,3-dioxepan-5-yl | Propyl | O | |
| 216 | 2-Methyl-1,3-dioxepan-5-yl | Ethyl | O | |
| 217 | 2-Methyl-1,3-dioxepan-5-yl | Propyl | O | |

Die Tetrahydropyranderivate der Formel I bzw. die sie enthaltenden herbiziden Mittel sind verträglich und somit selektiv in breitblättrigen Kulturen sowie in monokotylen Gewächsen, welche nicht zu den Gramineen zählen. Ferner sind Verbindungen darunter, welche sich auch in Gramineenkulturen, wie Weizen und Reis, selektiv verhalten und gleichzeitig unerwünschte Gräser bekämpfen.

Die Verbindungen der Formel I bzw. die sie enthaltenden herbiziden Mittel können beispielsweise in Form von direkt versprühbaren Lösungen, Pulvern, Suspensionen, auch hochprozentigen wäßrigen, öligen

oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen aus Mineralölfraktionen von mittlerem bis hohem Siedepunkt, wie Kerosin oder Dieselöl, ferner aus Kohlenteerölen sowie aus Ölen pflanzlichen oder tierischen Ursprungs, aus aliphatischen, cyclischen und aromatischen Kohlenwasserstoffen, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron, oder stark polare Lösungsmittel, wie N,N-Dimethylformamid, Dimethylsulfoxid, N-Methylpyrrolidon oder Wasser.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali- und Ammoniumsalze von aromatischen Sulfonsäuren, z.B. Lignin-, Phenol-, Naphthalin-und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetat, Sorbitester, Lignin, Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind:

I. Man vermischt 90 Gewichtsteile der Verbindung Nr. 51 mit 10 Gewichtsteilen N-Methyl-$\alpha$-pyrrolidon und erhält eine Lösung, die zur Anwendung in Form kleinster Tropfen geeignet ist.

II. 20 Gewichtsteile der Verbindung Nr. 52 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 36 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.

IV. 20 Gewichtsteile des Wirkstoffs Nr. 53 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige

Dispersion, die 0,02 Gew.% des Wirkstoffs enthält.

V. 20 Gewichtsteile des Wirkstoffs Nr. 54 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 71 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 36 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 51 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

Die Applikation der herbiziden Mittel bzw. der Wirkstoffe kann im Vorauflauf- oder vorzugsweise im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,01 bis 3, vorzugsweise 0,03 bis 1 kg/ha.

In Anbetracht des erfaßbaren Wirkungsspektrums zur Bekämpfung der unerwünschten Pflanzen der Verträglichkeit für Kulturpflanzen oder der erwünschten Beeinflussung des Wachstums derselben sowie angesichts der Vielfalt der Applikationsmethoden können die Verbindungen der Formel I sowie die sie enthaltenden herbiziden Mittel in einer großen Zahl von Kulturpflanzen eingesetzt werden.

In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |

| Botanischer Name | Deutscher Name |
|---|---|
| Cynodon dactylon | Bermudagras |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Helianthus tuberosus | Topinambur |
| Hevea brasiliensis | Parakautschukbaum |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |

| Botanischer Name | Deutscher Name |
|---|---|
| Sesamum indicum | Sesam |
| Solanum tuberosum | Kartoffel |
| Sorghum bicolor (S. vulgare) | Mohrenhirse |
| Spinacia oleracea | Spinat |
| Theobroma cacao | Kakaobaum |
| Triticum aestivum | Weizen |
| Triticum durum | Hartweizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Tetrahydropyranderivate der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäuren, Sulfonylharnstoffe, Imidazolinone, Aryloxyphenoxypropionsäurederiate und andere in Betracht.

Außerdem kann es von Nutzen sein, die Verbindungen der Formel I allein oder in Kombination mit anderen Herbiziden auch noch mit weiteren Pflanzenschutzmitteln gemischt gemeinsam aus zubringen, beispielsweise mit Mitteln zur Bekämpfung von Schädlingen oder phytopatogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate hergestellt werden.

Anwendungsbeispiele

Die Wirkung der Tetrahydropyranderivate der Formel Ia auf das Pflanzenwachstum läßt sich durch Gewächshausversuche zeigen:
Als Kulturgefäße dienen Plastikblumentöpfe mit 300 $cm^3$ Inhalt und lehmigem Sand mit etwa 3 % Humus als Substrat; zur Anzucht von Sojapflanzen wird Torf zugesetzt, um einen besseren Stand zu erzielen. Die Samen der Testpflanzen wurden nach Arten getrennt flach eingesät.

Bei Vorauflaufbehandlung werden die aufbereiteten Wirkstoffe unmittelbar danach auf die Erdoberfläche aufgebracht. Sie werden hierzu in Wasser als Verteilungsmittel suspendiert oder emulgiert und mittels fein verteilender Düsen gespritzt. Die Aufwandmenge beträgt 3.0 kg/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen auflaufen. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Zur Nachauflaufbehandlung werden entweder direkt gesäte und in den gleichen Gefäßen aufgewachsene Pflanzen ausgewählt, oder sie werden zuerst als Keimpflanzen getrennt angezogen und einige Tage vor der Behandlung in die Versuchsgefäße verpflanzt. Die Aufwandmengen für die Nachauflaufbehandlung betragen 0.125 und 0.03 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Man schätzt dabei visuell den Einfluß der Behandlung im Vergleich zur unbehandelten Kontrolle.
Die in den Gewächshausversuchen verwendeten Pflanzen setzen sich aus folgenden Arten zusammen:

| Latein. Name | Deutscher Name |
|---|---|
| Avena fatua | Flughafer |
| Avena sativa | Hafer |
| Alopecurus myosuroides | Ackerfuchsschwanz |
| Bromus inermis | Unbegrannte Trespe |
| Digitaria sanguinalis | Blutfingerhirse |
| Echinochloa crus-galli | Hühnerhirse |
| Glycine max. | Sojabohne |
| Lolium multiflorum | Ital. Raygras |
| Medicago sativa | Luzerne |
| Setaria italica | Kolbenhirse |
| Sorghum halepense | Mohrenhirse |
| Zea mays | Mais |

Der beispielhaft ausgewählte Wirkstoff Nr. 38 zeigt bei Anwendung 3,0 kg Wirkstoff/ha im Vorauflaufverfahren hohe herbizide Aktivität gegen grasartige Pflanzen. Im Nachauflaufverfahren lassen sich mit Wirkstoff Nr. 38 bereits mit geringen Aufwandmengen unerwünschte Pflanzen aus der Familie der Gramineen bekämpfen. Dabei wird die Kulturpflanze Luzerne nicht beeinträchtigt.

Wirkstoff Nr. 51 eignet sich im Nachauflaufverfahren mit 0,125 kg/ha zur Bekämpfung eines breiten Spektrums grasartiger Pflanzen. Soja als breitblättrige Beispielkultur bleibt völlig unbeeinflußt.

Die Wirkstoffe Nr. 36, 37, 51, 52, 53 und 54 zeigen bei Nachauflaufanwendung starke herbizide Wirkung gegen ein breites Spektrum unerwünschter Pflanzen aus der Familie der Gramineen, während Luzerne als Beispiel für eine breitblättrige Kulturpflanze völlig unbeeinflußt bleibt.

Die Wirkstoffe 36 und 71 zeigen z.B. bei Nachauflaufanwendung eine bessere Wirkung bei der Bekämpfung unerwünschter grasartiger Pflanzen als die in Tabelle 1 in GB-A-21 40 803 genannten Wirkstoffe Nr. 1 und Nr. 3.

## Patentansprüche

**1.** Tetrahydropyran-2,A-dione der Formel

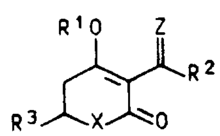

(I),

in der

- $R^1$ Wasserstoff, ein Kation, $C_1$-$C_{10}$-Alkylcarbonyl, $C_2$-$C_{10}$-Alkenylcarbonyl oder Benzoyl, das gegebenenfalls im Phenylring durch $C_1$-$C_8$-Alkyl substituiert ist,
- $R^2$ $C_1$-$C_5$-Alkyl,
- $R^3$ einen nichtaromatischen, gegebenenfalls durch $C_1$-$C_3$-Alkyl substituierten Heterocyclus mit 5 bis 7 Ringgliedern und maximal einer Doppelbindung im heterocyclischen Ring, der bis zu 2 Heteroatome aus der Gruppe Schwefel und Sauerstoff enthält,
- X Sauerstoff und
- Z Sauerstoff oder den Rest NO-$R^4$, wobei $R^4$ für $C_1$-$C_4$-Alkyl, $C_3$-$C_4$-Alkenyl, $C_3$-$C_4$-Alkinyl, $C_2$-$C_4$-Halogenalkyl, $C_2$-$C_4$-Halogenalkenyl, $C_2$-$C_3$-Alkoxyalkyl oder den Rest $CH_2$-$R^5$, in dem $R^5$ für einen 5-Ring-Heterocyclus mit bis zu 3 Heteroatomen, wobei die Zahl der Stickstoffatome maximal drei, die Zahl der Sauerstoffatome maximal zwei und die Zahl der Schwefelatome maximal eins beträgt, mit gegebenenfalls bis zu 2 Doppelbindungen und bis zu 2 Substituenten aus der Gruppe $C_1$-$C_4$-Alkyl, $C_1$-$C_4$-Alkoxy, Halogen, Trifluormethyl, $C_1$-$C_4$-Alkoxymethyl, $C_1$-$C_4$-Alkylthiomethyl und Vinyl oder für gegebenenfalls durch bis zu 3 Substituenten aus der Gruppe $C_1$-$C_4$-Alkyl, $C_1$-$C_4$-Alkoxy, Halogen, Trifluormethyl. Nitro und $C_1$-$C_4$-Dialkylamino substituiertes Phenyl steht, bedeuten.

2. Tetrahydropyran-2,4-dione der Formel I gemäß Anspruch 1, dadurch gekennzeichnet, daß $R^2$ in Formel I $C_2$-$C_3$-Alkyl bedeutet.

3. Tetrahydropyran-2,4-dione der Formel I gemäß Anspruch 1, dadurch gekennzeichnet, daß $R^3$ einen nichtaromatischen, gegebenenfalls durch $C_1$-$C_3$-Alkyl substituierten Heterocyclus mit 6 Ringgliedern bedeutet.

4. Tetrahydropyran-2,4-dione der Formel I gemäß Anspruch 1, dadurch gekennzeichnet, daß $R^3$ einen Tetrahydropyranyl- oder Tetrahydrothiopyranylrest bedeutet.

5. Verfahren zur Herstellung von Tetrahydropyran-2,4-dionen der Formel I gemäß Anspruch 1, in der Z Sauerstoff bedeutet, dadurch gekennzeichnet, daß man ein Tetrahydropyran-2,4-dion der Formel

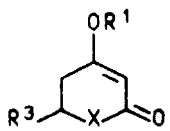

(II),

in der $R^1$, $R^3$ und X die in Anspruch 1 genannten Bedeutungen haben, zunächst mit einem Säurechlorid der Formel $R^2$-COCl, in der $R^2$ die in Anspruch 1 genannten Bedeutungen hat, in Gegenwart einer Base zum Enolester umsetzt und diesen anschließend mit einem sauren oder basischen Katalysator behandelt.

6. Verfahren zur Herstellung von Tetrahydropyran-2,4-dionen der Formel I gemäß Anspruch 1, in der Z den Rest NO-$R^4$ bedeutet, dadurch gekennzeichnet, daß man ein Tetrahydropyran-2,4-dionderivat der Formel I gemäß Anspruch 1, in der Z Sauerstoff bedeutet,
   a) mit einer Ammoniumverbindung der Formel $R^4$-O-$^{\oplus}H_3$-$Y^{\ominus}$, in der $R^4$ die in Anspruch 1 genannten Bedeutungen hat und Y ein Anion bedeutet, in einem inerten Verdünnungsmittel in Gegenwart einer Base und gegebenenfalls in Gegenwart von Wasser bei einer Temperatur zwischen 0 und 80 °C oder
   b) mit einem gegebenenfalls in wäßriger Lösung vorliegenden Hydroxylamin der Formel $R^4O$-$NH_2$, in der $R^4$ die in Anspruch 1 genannten Bedeutungen hat, in einem inerten Lösungsmittel umsetzt.

7. Herbizides und pflanzenwachstumsregulierendes Mittel, enthaltend ein Tetrahydropyran-2,4-dion der Formel I gemäß Anspruch 1.

8. Herbizides und pflanzenwachstumsregulierendes Mittel, enthaltend inerte Zusatzstoffe und ein Tetrahydropyran-2,4-dion der Formel I gemäß Anspruch 1.

9. Verfahren zur Bekämpfung von unerwünschtem Pflanzenwuchs, dadurch gekennzeichnet, daß man die unerwünschten Pflanzen und/oder die von unerwünschtem Pflanzenwachstum freizuhaltende Fläche mit einer herbizid wirksamen Menge eines Tetrahydropyran-2,4-dionderivates der Formel I gemäß Anspruch 1, in der Z den Rest NO-$R^4$ bedeutet, behandelt.

10. Verfahren zur Regulierung des Pflanzenwachstums, dadurch gekennzeichnet, daß man ein Tetrahydropyran-2,4-dion der Formel I gemäß Anspruch 1, in der Z Sauerstoff bedeutet, auf die Pflanzen und/oder deren Lebensraum einwirken läßt.

## Claims

1. A tetrahydropyran-2,4-dione of the formula

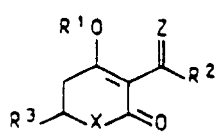

(I),

where $R^1$ is hydrogen, a cation, $C_1$-$C_{10}$-alkylcarbonyl,$C_2$-$C_{10}$-alkenylcarbonyl, or benzoyl which is unsubstituted or substituted in the phenyl ring by $C_1$-$C_8$-alkyl, $R^2$ is $C_1$-$C_5$-alkyl, $R^3$ is a non-aromatic, unsubstituted or $C_1$-$C_3$-alkyl-substituted heterocyclic radical of 5 to 7 ring members and having at most one double bond in the heterocyclic ring which contains up to two heteroatoms selected from the group consisting of sulfur and oxygen, X is oxygen, and Z is oxygen or the radical NO-$R^4$, $R^4$ being $C_1$-$C_4$-alkyl, $C_3$-$C_4$-alkenyl, $C_3$-$C_4$-alkynyl, $C_2$-$C_4$-haloalkyl, $C_2$-$C_4$-haloalkenyl, $C_2$-$C_3$-alkoxyalkyl or the radical $CH_2$-$R^5$, where $R^5$ is a heterocyclic radical of 5 ring members and containing from 1 to 3 heteroatoms, the number of nitrogen atoms being at most three, the number of oxygen atoms being at most two and the number of sulfur atoms being at most one, said heterocyclic radical containing from 0 to 2 double bonds and bearing either no substituents or one or two substituents selected from the group consisting of $C_1$-$C_4$-alkyl, $C_1$-$C_4$-alkoxy, halogen, trifluoromethyl, $C_1$-$C_4$-alkoxymethyl, $C_1$-$C_4$-alkylthiomethyl and vinyl, or $R^5$ is phenyl which is unsubstituted or bears from one to three substituents selected from the group consisting of $C_1$-$C_4$-alkyl, $C_1$-$C_4$-alkoxy, halogen, trifluoromethyl, nitro and $C_1$-$C_4$-dialkylamino.

**2.** A tetrahydropyran-2,4-dione of the formula I as claimed in claim 1, wherein $R^2$ in the formula I is $C_2$-$C_3$-alkyl.

**3.** A tetrahydropyran-2,4-dione of the formula I as claimed in claim 1, wherein $R^3$ is a non-aromatic 6-membered heterocyclic radical which is unsubstituted or substituted by $C_1$-$C_3$-alkyl.

**4.** A tetrahydropyran-2,4-dione of the formula I as claimed in claim 1, wherein $R^3$ is tetrahydropyranyl or tetrahydrothiopyranyl.

**5.** A process for the preparation of a tetrahydropyran-2,4-dione of the formula I as claimed in claim 1, where Z is oxygen, wherein a tetrahydropyran-2,4-dione of the formula

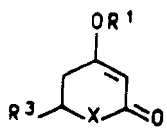

(II),

where $R^1$, $R^3$ and X have the meanings stated in claim 1, is first reacted with an acyl chloride of the formula $R^2$-COCl, where $R^2$ has the meanings stated in claim 1, in the presence of a base to give the enol ester, and the latter is then treated with an acidic or basic catalyst.

**6.** A process for the preparation of a tetrahydropyran-2,4-dione of the formula I as claimed in claim 1, where Z is NO-$R^4$, wherein a tetrahydropyran-2,4-dione derivative of the formula I as claimed in claim 1, where Z is oxygen,

a) is reacted with an ammonium compound of the formula $R^4$-O-$^{\oplus}NH_3$-$Y^{\ominus}$, where $R^4$ has the meanings stated in claim 1 and Y is an anion, in an inert diluent in the presence of a base and in the presence or absence of water at from 0 to 80 ° C, or

b) with a hydroxylamine of the formula $R^4O$-$NH_2$, where $R^4$ has the meanings stated in claim 1, which may be present in agueous solution, in an inert solvent.

**7.** A herbicidal and plant growth-regulating agent containing a tetrahydropyran-2,4-dione of the formula I as claimed in claim 1.

**8.** A herbicidal and plant growth-regulating agent containing inert additives and a tetrahydro-pyran-2,4-dione of the formula I as claimed in claim 1.

**9.** A method for controlling the growth of undesirable plants, wherein the undesirable plants and/or the area to be kept free from undesirable plant growth are treated with a herbicidally effective amount of a tetrahydropyran-2,4-dione derivative of the formula I as claimed in claim 1, where Z is NO-$R^4$.

**10.** A method for regulating plant growth, wherein a tetrahydropyran-2,4-dione of the formula I as claimed in claim 1, where Z is oxygen, is allowed to act on the plants and/or their habitat.

## Revendications

**1.** Tétrahydropyranne-2,4-diones de formule

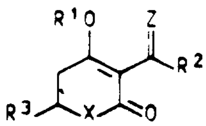

(I),

dans laquelle
$R^1$ représente l'hydrogène, un cation, un groupe (alkyle en C1-C10)-carbonyle, (alcényle en C2-C10)-carbonyle ou benzoyle éventuellement substitué dans le noyau phényle par un groupe alkyle en C1-C8,
$R^2$ représente un groupe alkyle en C1 -C5,
$R^3$ représente un hétérocycle non aromatique, éventuellement substitué par un groupe alkyle en C1-C3, contenant 5 à 7 chaînons et au maximum une double liaison dans l'hétérocycle, et qui contient jusqu'à 2 hétéroatomes choisis parmi le soufre et l'oxygène,
X représente l'oxygène, et
Z représente l'oxygène ou le groupe NO-$R^4$ dans lequel $R^4$ représente le groupe alkyle en C1-C4, alcényle en C3-C4, alcynyle en C3-C4, halogénoalkyle en C2-C4, halogénoalcényle en C2-C4, alcoxyalkyle en C2-C3 ou le groupe $CH_2$-$R^5$ dans lequel $R^5$ représente un hétérocycle à 5 chaînons contenant jusqu'à 3 hétéroatomes, le nombre des atomes d'azote étant au maximum de 3, le nombre des atomes d'oxygène au maximum de 2 et le nombre des atomes de soufre au maximum de 1, avec le cas échéant jusqu'à 2 doubles liaisons et jusqu'à 2 substituants choisis parmi les groupes alkyle en C1-C4, alcoxy en C1-C4, les halogènes, les groupes trifluorométhyle, (alcoxy en C1-C4)-méthyle, (alkyle en C1-C4)-thiométhyle et vinyle, ou bien un groupe phényle portant le cas échéant jusqu'à 3 substituants choisis parmi les groupes alkyle en C1-C4, alcoxy en C1-C4, les halogènes, les groupes trifluorométhyle, nitro et di-(alkyle en C1-C4)-amino.

**2.** Tétrahydropyranne-2,4-diones de formule I de la revendication 1, caractérisées en ce que $R^2$, dans la formule I, représente un groupe alkyle en C2-C3.

**3.** Tétrahyropyranne-2,4-diones de formule I de la revendication 1, caractérisées en ce que $R^3$ représente un hétérocycle non aromatique à 6 chaînons éventuellement substitué par un groupe alkyle en C1-C3.

**4.** Tétrahydropyranne-2,4-diones de formule I de la revendication 1, caractérisées en ce que $R^3$ représente un groupe tétrahydropyrannyle ou tétrahydrothiopyrannyle.

**5.** Procédé de préparation des tétrahydropyranne-2,4-diones de formule I de la revendication 1 dans laquelle Z représente l'oxygène, caractérisé en ce que l'on fait réagir une tétrahydropyranne-2,4-dione de formule

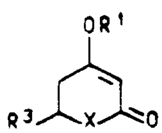

(II),

dans laquelle $R^1$, $R^3$ et X ont les significations indiquées dans la revendication 1, d'abord avec un chlorure d'acide de formule $R^2$-COCl dans laquelle $R^2$ a les significations indiquées dans la revendication 1, en présence d'une base, ce qui donne l'ester d'énol qu'on traite ensuite par un catalyseur acide ou basique.

**6.** Procédé de préparation des tétrahydropyranne-2,4-diones de formule I de la revendication 1 dans laquelle Z représente le groupe NO-$R^4$, caractérisé en ce que l'on fait réagir un dérivé de tétrahydropyranne-2,4-dione de formule I de la revendication 1 dans laquelle Z représente l'oxygène,

a) avec un dérivé d'ammonium de formule $R^4$-O-$^{\oplus}NH_3$-$Y^{\ominus}$, dans laquelle $R^4$ a les significations indiquées dans la revendication 1 et Y représente un anion, dans un diluant inerte en présence

d'une base et le cas échéant en présence d'eau à une température de 0 à 80 degrés C, ou bien
b) avec une hydroxylamine, éventuellement en solution aqueuse, de formule $R^4$-O-$NH_2$ dans laquelle $R^4$ a les significations indiquées dans la revendication 1, dans un solvant inerte.

**7.** Produit herbicide et régulateur de la croissance des végétaux contenant une tétrahydropyranne-2,4-dione de formule I de la revendication 1.

**8.** Produit herbicide et régulateur de la croissance des végétaux contenant des additifs inertes et une tétrahydropyranne-2,4-dione de formule I de la revendication 1.

**9.** Procédé pour combattre les croissances de végétaux indésirables, caractérisé en ce que l'on traite les végétaux indésirables et/ou la surface à protéger contre les croissances de végétaux indésirables par une quantité herbicide efficace d'un dérivé de tétrahydropyranne-2,4-dione de formule I de la revendication 1 dans laquelle Z représente le groupe NO-$R^4$.

**10.** Procédé pour la régulation de croissance des végétaux, caractérisé en ce que l'on fait agir sur les végétaux et/ou leur habitat une tétrahydropyranne-2,4-dione de formule I de la revendication 1, dans laquelle Z représente l'oxygène.